(19) Europäisches Patentamt
European Patent Office
Office européen des brevets

(11) **EP 3 352 644 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**31.07.2024   Patentblatt 2024/31**

(45) Hinweis auf die Patenterteilung:
**06.11.2019   Patentblatt 2019/45**

(21) Anmeldenummer: **16777577.4**

(22) Anmeldetag: **22.09.2016**

(51) Internationale Patentklassifikation (IPC):
**A61B 3/028** *(2006.01)*       **A61B 3/00** *(2006.01)*
**A61B 3/032** *(2006.01)*       **A61B 3/04** *(2006.01)*

(52) Gemeinsame Patentklassifikation (CPC):
**A61B 3/0041; A61B 3/032; A61B 3/04**

(86) Internationale Anmeldenummer:
**PCT/EP2016/072607**

(87) Internationale Veröffentlichungsnummer:
**WO 2017/050935 (30.03.2017 Gazette 2017/13)**

(54) **VERFAHREN UND SYSTEM ZUR BESTIMMUNG DER SUBJEKTIVEN REFRAKTIONSEIGENSCHAFTEN EINES AUGES**

METHOD AND SYSTEM FOR DETERMINING THE SUBJECTIVE REFRACTION PROPERTIES OF AN EYE

PROCÉDÉ ET DISPOSITIF POUR DÉTERMINER LES PROPRIÉTÉS DE RÉFRACTION SUBJECTIVES D'UN  OEIL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.09.2015   DE 102015116110**

(43) Veröffentlichungstag der Anmeldung:
**01.08.2018   Patentblatt 2018/31**

(73) Patentinhaber: **Carl Zeiss Vision International GmbH
73430 Aalen (DE)**

(72) Erfinder:
• **OHLENDORF, Arne
  72072 Tuebingen (DE)**
• **WAHL, Siegfried
  73072 Donzdorf (DE)**
• **CABEZA GUILLÉN, Jesús-Miguel
  73434 Aalen (DE)**

(74) Vertreter: **Glawe, Delfs, Moll
Partnerschaft mbB von
Patent- und Rechtsanwälten
Rothenbaumchaussee 58
20148 Hamburg (DE)**

(56) Entgegenhaltungen:
EP-A1- 2 842 479            WO-A2-2007/026368
WO-A2-2007/026368      DE-A1- 102004 055 754
DE-A1- 19 633 062          US-A- 4 929 076
US-A1- 2012 154 742      US-B2- 6 761 454

• **TOLHURST, D. J. ET AL.: "Amplitude spectra of natural images", OPHTHALMIC AND PHYSIOLOGICAL OPTICS, vol. 12, no. 2, 1992, pages 229 - 232, DOI: 10.1111/opo.12251**
• **HAUN A. M., PELI E.: "Perceived contrast in complex images", JOURNAL OF VISION, vol. 13, no. 13, 4 November 2013 (2013-11-04), pages 3 - 3, DOI: 10.1167/13.13.3**
• **MASAOKA K ET AL: "Sensation of Realness From High-Resolution Images of Real Objects", IEEE TRANSACTIONS ON BROADCASTING, IEEE SERVICE CENTER, PISCATAWAY, NJ, US, vol. 59, no. 1, 9 January 2013 (2013-01-09), pages 72 - 83, XP011520710, ISSN: 0018-9316, DOI: 10.1109/TBC.2012.2232491**

**Beschreibung**

Die vorliegende Erfindung betrifft ein System zur Bestimmung der subjektiven Refraktionseigenschaften eines Auges eines Probanden basierend auf der Verwendung eines natürlichen Bildes. Die vorliegende Erfindung betrifft ferner Verfahren zur Bestimmung der subjektiven Refraktionseigenschaften eines Auges eines Probanden basierend auf der Verwendung eines natürlichen Bildes bzw. einer natürlichen Szenerie, sowie die Verwendung eines natürlichen Bildes zur Bestimmung der subjektiven Refraktionseigenschaften eines Auges eines Probanden.

Zur subjektiven Refraktionsbestimmung sind Untersuchungsgeräte wie Messbrille oder Phoropter bekannt, bei denen beispielsweise sphärische oder zylindrische Probiergläser vor das Auge eines Probanden geschwenkt werden, um anhand der Angaben des Probanden seine Fehlsichtigkeit zu bestimmen.

Bei der subjektiven Refraktionsbestimmung gemäß nationalen sowie internationalen Normen kommen stets definierte Sehzeichen, auch Optotypen genannt, zum Einsatz. Die Optotypen werden schwarz auf weißem Grund mit hohem Kontrast dargestellt. Die Strichstärke (1/5 der Typengröße) des als genormtes DIN-Sehzeichen geltenden Landoltringes ist so bemessen, dass sie in einer für Sehschärfe 1 vorgesehenen Reihe dem Auge unter einem Winkel von 1 Bogenminute erscheint. Weitere Sehzeichen sind der Snellen-Haken, auch E-Haken genannt, der häufig für die Untersuchung von Kindern verwendet wird. Zudem wird bei Prüfungen des Nahvisus häufig eine Texttafel verwendet, zum Beispiel sogenannte Nieden-Leseproben. Die Optotypen werden einzeln oder in Reihen von 5 bzw. 10 Optotypen dargestellt.

Zur Refraktionsbestimmung werden dem Probanden nacheinander in einer definierten Entfernung Optotypen verschiedener Größen dargeboten. Der Proband sitzt hierfür in einem vorgegebenen Abstand von den angezeigten Optotypen. Die Fehler der Augen werden durch Annäherung an die Schwelle des Auflösungsvermögens des Probanden bestimmt.

Die Druckschrift US 6,325,513 beschreibt es als im Stand der Technik nachteilig, dass der Proband bei einer derartigen Messung keine entspannte Haltung einnehme und ein natürlicher Seheindruck verfälscht werde. In der Druckschrift wird daher eine Art kompakte Messbrille vorgeschlagen, bei welcher die Zeichen zur Refraktionsbestimmung auf die Retina projiziert werden. Mit der darin vorgeschlagenen Lösung werde eine Überlagerung erzeugter Messbilder mit der Umgebung bei bequemer, entspannter Sitzhaltung ermöglicht. Die subjektive Refraktionsbestimmung erfolgt anhand der Optotypen.

Die Druckschrift WO 2017/050935 A1 beschreibt ein multifunktionales optometrisches bzw. ophthalmologisches System zum Testen, Diagnostizieren oder zur Behandlung des Sehvermögens oder der Augen eines Subjekts. Das System weist ferner eine Anzeigevorrichtung in Form eines Mikro-Displays sowie eine Optikanordnung auf.

Die wissenschaftliche Veröffentlichung von Masaoka et al. "Sensation of Realness From High-Resolution Images of Real Objects", befasst sich mit dem Wirklichkeits- bzw. Echtheitsempfinden ("Sensation of Realness") von Bildern befasst.

Die Druckschrift DE 10 2004 055 754 A1 beschreibt ein Verfahren zur Durchführung eines Kontrastsehtests bei einer Testperson zur Bestimmung einer Kontrastsensitivitätsfunktion.

Die Druckschrift DE 4091126 C2 offenbart eine Vorrichtung zur binokularen Sehprüfung mit einer Anzeigevorrichtung auf der abwechselnd Sehtestzeichen für das linke und das rechte Auge darstellbar sind. Hierfür ist eine steuerbare Verschlusseinrichtung vorgesehen, die derart angesteuert wird, dass einige der dargestellten Sehtestzeichen nur dem linken Auge, einige der dargestellten Sehtestzeichen nur dem rechten Auge und einige der Sehtestzeichen beiden Augen dargeboten werden. Dadurch wird eine binokulare Sehprüfung ermöglicht. Die Anzeigevorrichtung kann ein Fernsehmonitor mit ausreichen hoher Bildwiederholfrequenz sein. Ein Fernsehmonitor weist jedoch meist keine ausreichende hohe Leuchtdichte auf, um die getrennten Sehzeichen dann für jedes Einzelauge mit den geforderten Leuchtdichten (beispielsweise 250cd/m$^2$) darzustellen.

EP 2 842 479 A1 offenbart Mittel und Verfahren zum Nachweis der Wirkungen von Zylindern mit geringer Astigmatismuskorrektur.

Vor diesem Hintergrund ist es eine Aufgabe der vorliegenden Erfindung ein System und ein Verfahren zur Bestimmung der subjektiven Refraktionseigenschaften eines Auges eines Probanden bereitzustellen, welche die Refraktionsbestimmung weiter verbessern und insbesondere eine aussagekräftige Bestimmung der habituellen Refraktionsfehler ermöglichen.

Die beanspruchten Gegenstände sind in den unabhängigen Ansprüchen definiert. Vorteilhafte Ausgestaltungen sind in den abhängigen Ansprüchen beschrieben.

Gemäß einem ersten Aspekt der Erfindung wird vorgeschlagen, ein System zur Bestimmung der subjektiven Refraktionseigenschaften eines Probanden gemäß Anspruch 1 bereitzustellen.

Gemäß einem weiteren Aspekt der Erfindung wird vorgeschlagen, ein Verfahren zur Bestimmung der subjektiven Refraktionseigenschaften eines Probanden gemäß Anspruch 13 bereitzustellen.

Die Erfinder haben erkannt, dass die bisherige Darstellung von Sehzeichen bei der Bestimmung der habituellen Refraktion nicht notwendigerweise zu einem optimalen Ergebnis führt. Bisherige Optotypen gemäß nationalen sowie internationalen Normen, die bei der Messung der Refraktionsfehler der Augen unter monokularen bzw. binokularen Bedingungen sowie während der MKH (Mess- und Korrektionsmethode nach Haase) genutzt werden, werden entsprechend der jeweiligen Normen präsentiert. Diese standar disierten Bedingungen entsprechen jedoch im Allgemeinen nicht denjenigen Bedingungen, welche ein Proband im Alltag vorfinden würde.

Im Rahmen der vorliegenden Offenbarung wird vorgeschlagen, ein natürliches Bild zur subjektiven Refraktionsbestimmung einzusetzen, insbesondere eine natürliche Szenerie wie beispielsweise eine Landschaftsaufnahme. Dabei zeichnet sich ein natürliches Bild vorzugsweise durch eine aus den Kanten der im Bild dargestellten Objekte resultierende Ortsfrequenzverteilung und vorzugsweise einen ortsfrequenzabhängigen Kontrast aus, wobei der Kontrast insbesondere mit zunehmender Ortsfrequenz abnimmt. Der Kontrastumfang eines im Rahmen der vorliegenden Erfindung verwendeten natürlichen Bildes, d.h. der Bereich der in dem natürlichen Bild vorhandenen Kontraste, beträgt bei einer Bestimmung des Kontrasts nach Michelson vorzugsweise von einschließlich 0,2 bis einschließlich 1, vorzugsweise einschließlich 0,3 bis einschließlich 1, vorzugsweise von einschließlich 0,4 bis einschließlich 1.

Die Ortsfrequenz gibt an, wie häufig sich sinusförmige Bestandteile eines Bildes wiederholen. Ist der Abstand zwischen den sinusförmigen Bestandteilen eines Bildes sehr groß, entspricht dies einer niederen Ortsfrequenz und einer groben Struktur. Ist im Gegensatz dazu der Abstand zwischen den sinusförmigen Bestandteilen gering, entspricht dies einer hohen Ortsfrequenz und einem feinen Detail. Wird der Abstand zur Anzeigevorrichtung verändert, ändern sich auch die spezifischen Ortsfrequenzen des betrachteten Bildes. Es versteht sich also, dass eine Ortsfrequenz bezogen auf eine Länge bzw. Pixel eines Bildschirms definierter Größe von dem Beobachtungsabstand d von der Anzeigeeinrichtung abhängen. Die Ortsfrequenz kann jedoch umgerechnet und als entfernungsunabhängiger Wert in Perioden pro Grad, auch als cpd ('cycles per degree') bezeichnet, angegeben werden. Unter Ortsfrequenz kann die Anzahl der Kanten pro Grad Sehwinkel verstanden werden.

Ein Bild kann neben seiner räumlichen Darstellung, beispielsweise in Form einer Matrix aus Pixeln unterschiedlicher Grau- bzw. Farbwerte, auch durch die darin enthaltenen räumlichen Frequenzen bzw. Ortsfrequenzen beschrieben werden. Vereinfacht gesprochen entspricht eine kleine Ortsfrequenz einer größeren Struktur. Eine hohe Ortsfrequenz entspricht einer kleineren Struktur. Die Ortsfrequenz ist somit ein Maß für die Größe einer abgebildeten Struktur.

Ferner besteht bei der Verwendung von Optotypen der Wunsch des Probanden stets das richtige Ergebnis zu nennen. Es entsteht eine Art Prüfungssituation, bei welcher der Proband sich möglichweise selbst unter Druck setzt. Der Lösungsraum der richtigen Antworten ist bei Sehzeichen, wie beispielsweise dem Snellen-Haken mit vier möglichen Orientierungen, begrenzt. Der Proband kann die Augen kurzfristig zusammenkneifen, um so das Erkennen des richtigen Ergebnisses zu forcieren.

Ferner kann es sein, dass ein Proband nicht seine gewohnten Kopf- und Körperhaltung einnimmt sondern unnatürlich aufrecht oder angespannt sitzt. Auch dies kann zur Folge haben, dass Seheindrücke anders wahrgenommen werden und sich im schlechtesten Fall negativ auf die ermittelte Stärke auswirken. Es können sich somit Abweichungen zwischen den Ergebnissen einer subjektiven Refraktionsbestimmung und den habituellen Refraktionsfehlern des Probanden ergeben.

Bei der hierin beschriebenen Lösung wird daher vorgeschlagen, die Bestimmung der subjektiven Refraktionseigenschaften des Auges des Probanden anhand natürlicher Bilder mit definierten Merkmalen durchzuführen. Natürliche Bilder können dabei Fotografien oder Abbildungen einer natürlichen Szenerie, insbesondere einer typischen Umwelt, in der man lebt, entsprechen. Die Refraktionsbestimmung kann anhand der auf den natürlichen Bildern gezeigten Inhalte auf die alltäglichen Sehbedingungen des Probanden zugeschnitten werden. Ein weiterer Vorteil dieser Lösung gegenüber Sehzeichen ist, dass der Proband nicht von vorneherein weiß, welche Antwort von ihm erwartet wird.

Basierend auf den natürlichen Bildern können die habituellen Refraktionsfehler der Augen durch Annäherung an die Schwelle des Auflösungsvermögens bestimmt werden. Hierzu weist das natürliche Bild verschiedene Merkmale unterschiedlicher Strukturgrößen bzw. Ortsfrequenzen auf. Aus dem vorgegebenen Abstand von der Anzeigevorrichtung betrachtet entsprechen die Merkmale unterschiedlicher Größe unterschiedlichen Betrachtungswinkeln, welche wiederum zur Bestimmung des Auflösungsvermögens herangezogen werden. Beispielsweise kann getestet werden, ob der Proband bzw. mit welchen lichtbrechenden Elementen im optischen Pfad der Proband eine Struktur wie beispielsweise einen Baum oder eine Felsformation erkennen kann. Entsprechendes gilt für eine natürliche Szenerie einer Testumgebung.

Die Optikanordnung dient dazu verschiedene lichtbrechende Elemente wie beispielsweise sphärische oder zylindrische Probiergläser in den optischen Pfad zwischen dem Auge des Probanden und der Anzeigevorrichtung einzubringen. Die Optikanordnung kann beispielsweise ein Phoropter oder eine Messbrille sein.

Die vorstehend für den ersten Aspekt der Erfindung ausführlich beschriebenen Vorteile gelten für die weiteren Aspekte der Erfindung entsprechend.

Mit den vorgeschlagenen Lösungen kann die Refraktionsbestimmung weiter verbessert werden, indem eine habituelle Refraktionsbestimmung unter Bedingungen durchgeführt werden kann, die den natürlichen Sehbedingungen des Probanden eher entsprechen.

Die eingangs gestellte Aufgabe wird daher vollkommen gelöst.

In einer Ausgestaltung des Systems kann das natürliche Bild oder ein Bildbereich des natürlichen Bildes einen ortsfrequenzabhängigen Kontrast aufweisen, wobei der Kontrast mit zunehmender Ortsfrequenz abnimmt.

Mit anderen Worten kann somit zumindest ein Bereich des natürlichen Bildes bzw. Bildes zur Bestimmung der subjektiven Refraktionseigenschaften des Auges einen Kontrast aufweisen, welcher bei kleinen Ortsfrequenzen größer ist, als bei hohen Ortsfrequenzen. Eine kleine Ortsfrequenz entspricht dabei einer größeren Struktur. Eine hohe Ortsfrequenz entspricht dabei einer kleineren Struktur. In diesem Kontext kann sich der Ausdruck natürliches Bild auf ein Bild beziehen, welches zumindest einen Bildbereich mit ortsfrequenzabhängigem Kontrast aufweist, wobei der Kontrast mit zunehmender Ortsfrequenz abnimmt. Im Gegensatz hierzu weisen die Optotypen bei konventionellen Sehtesttafeln gemäß nationalen sowie internationalen Normen unabhängig von der Strukturgröße bzw. Ortsfrequenz einen konstanten Kontrast auf. Bei konventionellen Sehtesttafeln werden große wie kleine Optotypen üblicherweise schwarz auf weißen Grund dargestellt. Die Darstellung eines Optotypen auf einer konventionellen Sehtesttafel weist somit unabhängig von der Ortsfrequenz einen konstanten Kontrast nach Michelson mit einem konstanten Wert zwischen 0,9 und 1 auf. Demgegenüber beträgt der Kontrastumfang eines im Rahmen der vorliegenden Erfindung verwendeten natürlichen Bildes bei einer Bestimmung des Kontrasts nach Michelson vorzugsweise von einschließlich 0,2 bis einschließlich 1, vorzugsweise einschließlich 0,3 bis einschließlich 1, vorzugsweise von einschließlich 0,4 bis einschließlich 1.

Vorzugsweise kann der Kontrast umgekehrt proportional zur Ortsfrequenz sein. Zumindest Abschnittsweise kann somit die Beziehung $K(f){\sim}1/f$ gelten oder allgemeiner $K(f){\sim}f^a$ mit $-1{,}5{\leq}a{\leq}{-}0{,}8$, insbesondere $a{=}{-}1{,}2$. Demgegenüber weisen konventionelle Sehtesttafeln, mit beispielsweise schwarzen Sehtestzeichen auf weißem Grund, einen bezüglich der Ortsfrequenz konstanten Kontrast auf. Hierdurch soll bei konventionellen Sehtesttafeln eine maximale Erkennbarkeit der Optotypen sichergestellt werden. Die Erfinder haben jedoch den unerwarteten Effekt erkannt, dass die Verwendung von natürlichen Bildern, einer natürlichen Szenerie bzw. Bildern zur Bestimmung der subjektiven Refraktionseigenschaften eines Auges, welche zumindest in Bereichen einen ortsfrequenzabhängigen Kontrast aufweisen, welcher mit zunehmender Ortsfrequenz abnimmt, zu einem Ergebnis führen kann, welches eine konventionelle Refraktionsbestimmung übertrifft. Insbesondere kann eine basierend auf einem solchen Ergebnis der Refraktionsbestimmung angepasste Sehhilfe von Probanden im Alltag als angenehmer empfunden werden.

Der Kontrast bzw. photometrische Kontrast kann als Leuchtdichteunterschied verstanden werden. Der Kontrast kann sich zwischen örtlich mehr oder weniger benachbarten Reizen bestimmt werden. Nach Michelson wird der Kontrast $K_M$ für Gittermuster definiert durch

$$K_M = \frac{L_{Max} - L_{Min}}{L_{Max} + L_{Min}} \qquad (1)$$

mit $L_{max}$ = Leuchtdichtemaximum und $L_{Min}$ = Leuchtdichteminimum. In diesem Falle variiert der Kontrast bei festgelegter, mittlerer Helligkeit durch Erhöhung oder Verringerung der Helligkeit um den gleichen Betrag. Werden jedoch kleine Sehobjekte, wie beispielsweise Landoltringe, verwendet, kann die Definition des Kontrastes nach Weber erfolgen durch

$$K_W = \frac{L_I - L_U}{L_U} \qquad (2)$$

mit $L_I$ = Leuchtdichte Innfeld und $L_U$ = Leuchtdichte Umfeld. Im Fall der Sehschärfeuntersuchung erfolgt die Definition des Kontrastes nach Weber. Angaben in dieser Offenbarung beziehen sich auf die Definition des Kontrastes nach Michelson (siehe auch Bex. et al. "Spatial frequency, phase, and the contrast of natural images", Journal of the Optical Society of America, Vol. 19, No. 6, 2002).

Vorzugsweise wird ein Kontrast des natürlichen Bildes für höhere Ortsfrequenzen angehoben, insbesondere auf ein Kontrastniveau einer durch das natürliche Bild abgebildeten natürlichen Szenerie.

Indem ein Kontrast für höhere Ortsfrequenzen angehoben wird, kann eine Tiefpasscharakteristik eines Abbildungssystems bei der Bildaufnahme des natürlichen Bildes kompensiert werden kann. Ein Vorteil dieser Ausgestaltung besteht darin, dass bei Betrachtung des natürlichen Bildes auf einer Anzeigevorrichtung ein ähnlicher Seheindruck hervorgerufen werden, als ob der Proband eine natürliche Szenerie direkt betrachten würde anstelle einer Abbildung der natürlichen Szenerie.

In einer Ausgestaltung des Systems kann vorgesehen sein, dass das natürliche Bild bzw. die darin abgebildete natürliche Szenerie eine Abbildung einer typischen Umwelt ist.

Ein Vorteil dieser Ausgestaltung ist, dass der Proband auf gewohnte Inhalte blickt und sich somit psychologisch besser von der Testsituation lösen kann. Der Proband nimmt somit mitunter eine entspanntere Haltung ein. Vorzugsweise können natürliche Bilder Abbildungen, insbesondere Fotografien oder fotorealistischen oder gerenderten Abbildungen, von der typischen Umwelt in der man lebt, entsprechen. Beispielsweise können für einen Probanden Landschaftsbilder gezeigt werden. Insbesondere kann ein natürliches Bild eine Fotografie oder fotorealistische Abbildungen einer Landschaftsszene, insbesondere eine zusammenhängende Darstellung einer Szene einer Landschaft, insbesondere mit landschaftstypischer Flora und Fauna. Es kann sich bei natürlichen Bildern somit um ein gewohntes Umfeld des Probanden handeln. Hiermit können bei der habituellen Refraktionsbestimmung mitunter bessere Ergebnisse erzielt werden, da eine übliche Sehsituation die Grundlage der Refraktionsbestimmung bildet. Ferner können natürliche Bilder derart gewählt werden, dass sie beim Probanden positive Assoziationen wecken, wie beispielsweise Bilder von einem Strand, Wald oder dergleichen.

In einer weiteren Ausgestaltung des Systems kann vorgesehen sein, dass das natürliche Bild Merkmale zur Bestimmung der subjektiven Refraktionseigenschaften aufweist.

Insbesondere kann in einer Ausgestaltung des Systems vorgesehen sein, dass das natürliche Bild mindestens zwei Bereiche mit unterschiedlichen Ortsfrequenzen aufweist. Bereiche unterschiedlicher Ortsfrequenzen, also unterschiedlicher räumlicher Frequenzen, dienen dazu die Sehschärfe zu prüfen. Um kleine Strukturen, also Strukturen mit hoher Ortfrequenz, auflösen zu können ist eine hohe Sehschärfe erforderlich. In einem natürlichen Bild können sogenannte "Regions of Interest" (ROIs) definiert werden, die einem bestimmten Bereich von Ortsfrequenzen zugehörig sind, welcher einer bestimmten Sehschärfe entspricht. Mit anderen Worten können ROIs somit Bereiche in einem natürlichen Bild darstellen, die einen abzuprüfenden Bereich von Ortsfrequenzen beinhalten. Fehler der Augen werden durch Annäherung an die natürliche Schwelle des Auflösungsvermögens bestimmt. Eine hohe Ortsfrequenz entspricht dabei einer kleinen Strukturgröße. Der Proband kann aufgefordert werden, Merkmale aus Bereichen mit unterschiedlichen, vorzugsweise mit immer größer werdenden Ortsfrequenzen, also kleineren Strukturgrößen oder Objekten, zu benennen, bis die Schwelle seines Auflösungsvermögens erreicht ist. Vorzugsweise nimmt der Kontrast für Bereiche mit zunehmenden Ortsfrequenzen ab. Insbesondere kann der Kontrast umgekehrt proportional zur Ortsfrequenz sein.

In einer weiteren Ausgestaltung des Systems kann vorgesehen sein, dass das natürliche Bild eine Verteilung von Ortfrequenzen aufweist, welche eine Mehrzahl, vorzugsweise alle, zur Bestimmung von subjektiven Refraktionseigenschaften des Auges des Probanden erforderlichen Ortsfrequenzen in einem einzigen Bild umfassen.

Mit anderen Worten weist das natürliche Bild vorzugsweise eine vorgegebene Verteilung von Ortsfrequenzen auf, die in Abhängigkeit der Detailtreue hoch ist bzw. mit abnehmender Detailtreue abnimmt. Somit können vorzugsweise alle Schwellen für die Bestimmung der Refraktionsfehler in einem Bild gleichzeitig präsentiert werden. Ein Vorteil dieser Ausgestaltung ist, dass nur ein einziges Bild zur Refraktionsbestimmung erforderlich ist. Beispielsweise kann eine Wolkenkratzerszene die Gebäude als größte Elemente, Fahrzeuge als mittlere Elemente bis hin zu Werbetafeln mit einzelnen Schriftzeichen als kleine Elemente aufweisen. Selbstverständliche sind weitere Zwischenstufen möglich. In einem weiteren Beispiel wird eine Naturszene gezeigt mit Bergketten bis hin zu Blättern oder Tannennadeln. Vorzugweise ist das natürliche Bild entgegen konventionellen Sehtesttafeln nicht derart aufgebaut, dass die Größe der Bildelemente in einer Richtung, beispielsweise von oben nach unten, immer kleiner wird. Insbesondere ist eine Größenabfolge nicht in einer Richtung monoton steigend oder fallend. Insbesondere kann die Verteilung von Ortsfrequenzen im Bild pseudozufällig sein. Die Verteilung unterliegt also keinem Schema, welches dem Probanden unmittelbar ersichtlich wäre. Optotypen bzw. Einzeloptotypen können optional, beispielsweise eigebettet in einer solchen Szene, augmentiert präsentiert werden.

In einer weiteren Ausgestaltung des Systems kann vorgesehen sein, dass das natürliche Bild Strukturen mit unterschiedlichen Ortsfrequenzen (f) zeigt, wobei die Ortsfrequenzverteilung, bei Betrachtung aus dem vorgegebenen Abstand, mindestens eine Ortsfrequenz kleiner oder gleich 0,3 und mindestens eine Ortsfrequenz größer oder gleich 60 Perioden pro Grad aufweist, vorzugsweise mindestens eine Ortsfrequenz kleiner oder gleich 0,01 und mindestens eine Ortsfrequenz größer oder gleich 80 Perioden pro Grad aufweist.

Die Ortsfrequenzverteilung wird vorzugsweise in Perioden pro Grad also als eine Ortsfrequenz hinsichtlich eines Winkels angegeben, da die Sehschärfe des Probanden als Maß für das Winkelauflösungsvermögen gesehen werden kann. Der Winkel wiederum kann aus dem vorgegebenen Abstand, aus welchem der Proband die Anzeigevorrichtung betrachtet und der Größe der auf der Anzeigevorrichtung angezeigten Struktur berechnet werden. Beispielsweise kann das natürliche Bild eine Ortsfrequenzverteilung von 0.009 bis 85 Perioden pro Grad aufweisen. In einem weiteren Beispiel kann das natürliche Bild eine Ortsfrequenzverteilung von 0.02 bis 70 Perioden pro Grad aufweisen. Es gilt also die Beziehung unterer Wert ≤ angegebener Bereich ≤ oberer Wert. Die Strukturen im Intervall dazwischen können eine beliebige Anzahl an Ortsfrequenzen zeigen. Unter einer Struktur mit einer Ortsfrequenz ist vorliegend eine Struktur mit einer der Ortsfrequenz entsprechenden räumlichen Größe zu verstehen.

In einer weiteren Ausgestaltung des Systems kann vorgesehen sein, dass das System ferner eine Auswahleinrichtung zum Auswählen des natürlichen Bildes gemäß Präferenzen des Probanden aufweist.

Ein Vorteil dieser Ausgestaltung ist, dass die Refraktionsbestimmung auf ein übliches Umfeld des Probanden abgestimmt werden kann, beispielsweise hinsichtlich Beleuchtung, Kontrast, Farbspektrum sowie der dargestellten Inhalte. Vorzugsweise kann somit die neuronale Transferfunktion des Probanden bei der Refraktionsbestimmung mit berücksichtigt werden. Beispielsweise kann eine für den Probanden natürliche Umgebung wie Stadt, Wald, Strand gewählt werden. Ein weiterer Vorteil kann darin bestehen, dass der Proband in der Messsituation entspannter sein ist und somit realistischere Werte der subjektiven Refraktionseigenschaften seines Auges erhalten werden können.

In einer weiteren Ausgestaltung des Systems kann vorgesehen sein, dass das natürliche Bild mindestens eine Verfremdung aufweist.

Beispielsweise kann es sich bei einer Verfremdung um eine computergraphische Veränderung handeln. Ein Vorteil dieser Ausgestaltung kann darin bestehen, dass einer Erwartungshaltung für das Erkennen von Details in natürlichen Bildern entgegentreten werden kann. Es können unerwartete Details angezeigt werden, wie beispielsweise eine bestimmte Form einer Wolke, Eichenblätter an einem Ahornbaum, oder eine unerwartete Form eines Baumstammes. Auch hierbei können die Präferenzen des Probanden vorteilhaft berücksichtigt werden, indem mit einer Erwartungshaltung des Probanden gespielt werden kann und gezielt für diesen Probanden unerwartete Verfremdungen vorgenommen werden können.

In einer weiteren Ausgestaltung des Systems kann vorgesehen sein, dass wenigstens ein Sehzeichen zumindest teilweise in das natürliche Bild integriert ist.

Vorzugsweise können Sehzeichen beziehungsweise deren Geometrien, die in nationalen bzw. internationalen Normen zur Bestimmung der subjektiven Refraktion angezeigt werden, ganz oder teilweise in das natürliche Bild mit aufgenommen sein. Zum Beispiel können Abstände oder Strichstärken von Merkmalen des natürlichen Bildes denen von normierten Sehzeichen entsprechen.

In einer weiteren Ausgestaltung des Systems kann vorgesehen sein, dass die Anzeigevorrichtung eine gekrümmte Anzeigefläche aufweist.

Ein gekrümmter Bildschirm ist von Vorteil, da dieser den anatomischen Bedingungen des Auges mit gekrümmter Netzhaut entspricht. Dennoch ist die Darstellung auf einem herkömmlichen, flachen Bildschirm auch möglich. Um eine Annäherung an die anatomischen Gegebenheiten des Auges zu ermöglichen, erfolgt die Darstellung der natürlichen Bildern vorzugsweise auf einer gekrümmten Anzeigeeinheit insbesondere mit einer Bildschirmdiagonale von 55 Zoll oder größer, um ein immersives Gefühl und damit einen natürlicheren Seheindruck bei der Refraktionsbestimmung zu erhalten. Die Verwendung von bisher üblichen Monitoren ist jedoch nicht ausgeschlossen. Auch die Darstellung über einen Beamer auf gerade oder gekrümmte Flächen im Raum ist denkbar. Auch der Einsatz eines Virtual-Reality-(VR-) Systems mit vorzugsweise großem Gesichtsfeld (Field of View) und einstellbarer Fokusfläche von nahen Abständen bis hin zu virtuellen Abständen im Fernbereich und optional integriertem Phoropter oder adaptiver Optik ist denkbar. Bei einer gekrümmten Anzeigefläche kann der Abstand zum Auge des Betrachters für jeden Punkt der Anzeigefläche individuell bestimmt werden. Zur Vereinfachung kann der Abstand zum Krümmungsmittelpunkt betrachtet werden. Auch eine Holobrille kann eingesetzt werden.

In einer weiteren Ausgestaltung des Systems kann vorgesehen sein, dass die Bestimmung der subjektiven Refraktionseigenschaften monokular im zweidimensionalen Raum erfolgt oder monokular unter binokularen Bedingungen.

Die Refraktionsbestimmung kann somit unter zweidimensionalen oder dreidimensionalen Bedingungen erfolgen. Bei der Refraktionsbestimmung unter binokularen Bedingungen im dreidimensionalen Raum können beispielsweise Polarisationsfilter zum Einsatz kommen, um die Beiträge für das rechte und das linke Auge zu separieren.

Das System weist Mittel zum Erfassen der Augenbewegung des Probanden auf.

Wird anspruchsgemäß ein Mittel zur Messung der Augenbewegungen in das System integriert, kann über die Analyse der Verteilung der Augenbewegungen auf dem gesehenen Bild vorzugweise in Echtzeit nachvollzogen werden, welche Ortsfrequenzen häufig angeblickt werden. Daraus kann abgeleitet werden, welche Ortsfrequenzen häufig erkannt werden. Dies wiederum kann zur genauen Definition der auflösbaren Schwelle des Auges mit und ohne Brille verwendet werden. Die subjektiven Refraktionseigenschaften des Auges können bestimmt werden, indem mit der Optikanordnung nacheinander verschiedene lichtbrechende Elemente (z.B. sphärische Linsen etc.) eingebracht werden und die auflösbare Schwelle des Auges jeweils bestimmt wird. Dies kann wiederholt werden, bis eine Korrektur einer Fehlsichtigkeit erreicht ist. Ein Vorteil dieser Ausgestaltung liegt darin, dass die Bestimmung von Refraktionsparametern des Probanden ohne seine ausdrückliche Rückmeldung erfolgen kann. Dies ist insbesondere von Vorteil bei Probanden, die sich nicht oder nicht adäquat artikulieren können, wie beispielsweise bei Kindern oder Probanden mit Einschränkungen. In einer weiteren Ausgestaltung des Systems kann vorgesehen sein, dass das System ferner Mittel zum Erfassen der Kopfbewegung des Probanden aufweist. Wenn die Kopfbewegungen des Probanden erfasst werden, erlaubt dies die Anpassung des anzublickenden Bildes an die Kopfbewegungen des Probanden in Echtzeit, während dessen Refraktion bestimmt wird.

In einer weiteren Ausgestaltung des Systems kann vorgesehen sein, dass das natürliche Bild ein bewegtes Bild bzw. Video ist.

Ein Vorteil dieser Ausgestaltung ist, dass die Messsituation aufgelockert werden kann, sodass die Refraktionsbestimmung mit einem entspannteren Probanden erfolgen kann. Die Ergebnisse entsprechen somit eher den natürlichen Sehbedingungen. Bei bewegten Bildern kann es sich um ein zweidimensionales oder auch dreidimensionales Video handeln, welches dem Probanden während der habituellen Refraktionsbestimmung präsentiert wird.

In einer weiteren Ausgestaltung des Systems kann vorgesehen sein, dass das System ferner einen exzentri-

schen Photorefraktor aufweist.

Während der Refraktionsbestimmung kann vorzugsweise eine kontinuierliche Messung der Refraktionsfehler beispielsweise mittels exzentrischer Photorefraktion erfolgen. Ein Untersucher kann dabei beispielsweise auf seinem Bildschirm die Restrefraktion des Auges verfolgen und die Qualität seiner Refraktion während der Bestimmung überprüfen. Der exzentrische Photorefraktor ist vorzugsweise in der gleichen Entfernung wie die Anzeigevorrichtung, auf der das natürliche Bild präsentiert wird, angeordnet. Alternativ kann der Photorefraktor bei oder als Teil der Optikanordnung vorgesehen sein. Die Optikanordnung kann somit einen exzentrischen Photorefraktor aufweisen.

Vorzugweise kann die Ermittlung der habituellen Refraktionsfehler mittels natürlicher Bilder in eine bekannte subjektive Methode integriert werden, wie beispielsweise mittels Messbrille, manuellem oder digitalem Phoropter.

Beispielsweise können ein Schritt oder mehrere der folgenden Schritte der Refraktionsbestimmung mit Bildinhalten aus natürlichen Bildern durchgeführt werden: Bestimmen eines besten sphärischen Glases, Bestimmen eines Astigmatismus, Achsabgleich eines Astigmatismus, Stärkenabgleich eines Astigmatismus und monokularer und/oder binokularer sphärischer Feinabgleich (rot/grün). Der Proband schaut dabei auf die Anzeigevorrichtung, auf welcher das natürliche Bild dargestellt wird. Um die habituelle Fehlsichtigkeit zu messen muss der Proband Bildinhalte bewerten, vorlesen bzw. erkennen, wie beispielsweise in einem Landschaftsbild Bäume, Äste oder Blätter, damit ein sphärischer und gegebenenfalls auch astigmatischer Fehler des Auges bestimmt und somit auch korrigiert werden kann. Ziel der Refraktionsbestimmung kann dabei die Korrektion der habituellen subjektiven Refraktionseigenschaften mit der maximal erkennbaren Ortsfrequenz bei maximal positiver Korrektur sein.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen. Insbesondere gelten die obigen Ausgestaltungen und Weiterbildungen neben natürlichen Bildern für die vorstehend beschriebenen weiteren Aspekte der Erfindung und auch für eine Testumgebung, insbesondere mit einer natürlichen Szenerie, entsprechend.

Ausführungsformen der Erfindung sind in der Zeichnung dargestellt und werden in der nachfolgenden Beschreibung näher erläutert. Es zeigen:

Fig. 1      eine Ausführungsform eines Systems zur Bestimmung der subjektiven Refraktionseigenschaften eines Auges eines Probanden basierend auf der Verwendung eines natürlichen Bildes,

Fig. 2      eine Ausführungsform eines entsprechenden Verfahrens,

Fig. 3      ein beispielhaftes Szenario zur Bestimmung der subjektiven Refraktionseigenschaften eines Auges eines Probanden anhand einer natürlichen Szenerie,

Fig. 4      eine Ausführungsform eines entsprechenden Verfahrens,

Fig. 5      eine Beispiel einer konventionellen Sehtesttafel,

Fig. 6      ein schematisches Diagramm des Kontrastes gegenüber der Ortsfrequenz für konventionelle Sehtesttafeln,

Fig. 7      ein Beispiel eines natürlichen Bildes,

Fig. 8      ein Ortsfrequenzdiagram des Beispielbildes aus Fig. 7,

Fig. 9      das Beispielbild aus Fig. 7 mit Kennzeichnung von Bereichen unterschiedlicher Ortsfrequenzen, und

Fig. 10     ein schematisches Diagramm des Kontrastes gegenüber der Ortsfrequenz für ein Bild gemäß der vorliegenden Offenbarung,

Fig. 11     eine Ausführungsform eines Verfahrens zur Bestimmung der subjektiven Refraktionseigenschaften eines Auges eines Probanden.

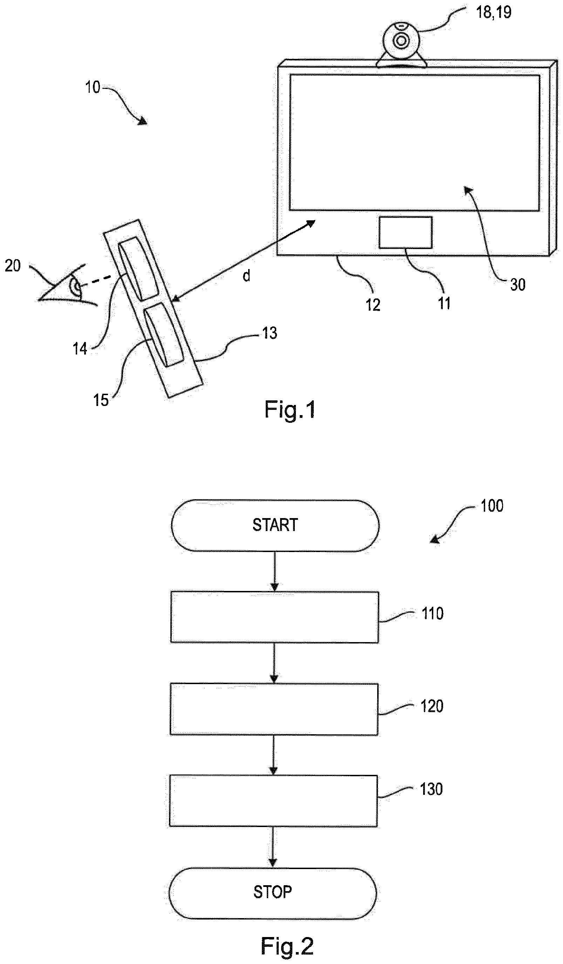

Fig. 1 zeigt eine Ausführungsform eines Systems zur Bestimmung der subjektiven Refraktionseigenschaften eines Auges eines Probanden basierend auf der Verwendung eines natürlichen Bildes. Das System ist dabei in seiner Gesamtheit mit Bezugszeichen 10 bezeichnet.

Das System 10 zur Bestimmung der subjektiven Refraktionseigenschaften des Auges 20 des Probanden weist dabei Folgendes auf: eine Speichereinrichtung 11, in welcher mindestens ein natürliches Bild 30 gespeichert ist; eine Anzeigevorrichtung 12 zum Anzeigen des mindestens einen natürlichen Bildes 30 aus der Speichereinrichtung 11; und eine Optikanordnung 13 zum Einstellen von verschiedenen lichtbrechenden Elementen 14, 15 in einen optischen Pfad zwischen dem Auge 20 des Probanden und der Anzeigevorrichtung 12, wobei die Optikanordnung 13 in einem vorgegebenen Abstand d von der Anzeigevorrichtung 12 angeordnet ist.

Die Speichereinrichtung 11 kann in dieser Ausführungsform in die Anzeigevorrichtung 12 integriert sein oder räumlich getrennt von der Anzeigevorrichtung 11 angeordnet und kabellos oder kabelgebunden mit dieser verbunden sein. Es ist lediglich erforderlich, dass das in der Speichereinrichtung 11 gespeicherte natürliche Bild 30 auf der Anzeigevorrichtung 12 angezeigt werden kann.

Bei der Anzeigevorrichtung 12 handelt es sich im vorliegenden Beispiel um einen Flachbildfernseher. Dieser weist vorzugsweise eine Bildschirmdiagonale von nicht weniger als 55 Zoll auf. Ferner kann die Anzeigefläche der Anzeigevorrichtung gekrümmt sein, um eine immersivere Darstellung des natürlichen Bildes 30 zu ermöglichen. Damit entspricht die Sehsituation bei der Refraktionsbestimmung eher den üblichen Sehbedingungen des Probanden in seiner üblichen Umgebung. Bei der Anzeigevorrichtung kann es sich um einen 3D-Bildschirm handeln. Ein Vorteil der dreidimensionalen Darstellung ist, dass die Bestimmung der subjektiven Refraktionseigenschaften nicht nur monokular im zweidimensionalen Raum erfolgen kann, sondern optional auch monokular unter bidirektionalen Bedingungen. Auch die Darstellung bewegter natürlicher Bilder in Form von Videosequenzen ist möglich. Für die 3D Darstellung können bekannte Techniken wie Shutter-Techniken oder Polarisationsfilter zum Einsatz kommen. Optional handelt es sich bei der Anzeigevorrichtung 12 um eine holographische Anzeige.

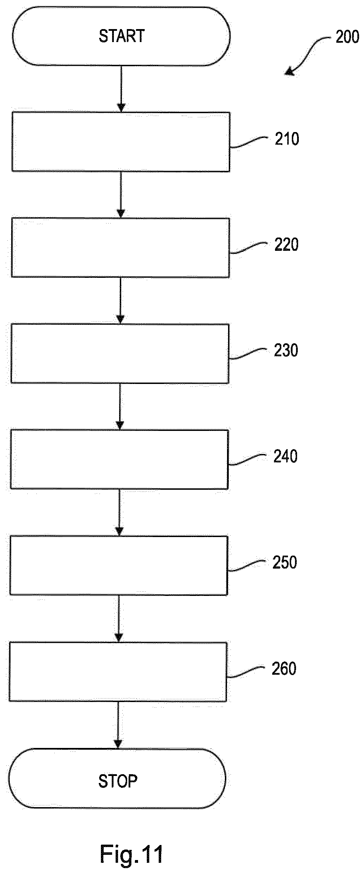

Bei der Optikanordnung handelt es sich im vorliegenden Beispiel um einen Phoropter, welcher schematisch durch zwei Linsenelemente 14 und 15 vereinfacht dargestellt ist. Alternativ kann beispielsweise eine Messbrille zu Einsatz kommen. Bei einem beispielhaften Szenario zur Refraktionsbestimmung setzt sich der Proband gegenüber der Anzeigevorrichtung 12 in einem definierten Abstand von der Anzeigevorrichtung 12 auf einen Untersuchungsstuhl und betrachtet das natürliche Bild 30, welches auf der Anzeigevorrichtung 12 präsentiert wird, durch die Optikanordnung 13. Ein beispielhafter Ablauf der Refraktionsbestimmung wird nachfolgend mit Bezugnahme auf Fig. 11 detaillierter beschrieben.

Des Weiteren kann optional vorgesehen sein, dass das System 10 Mittel 18 zum Erfassen der Kopfbewegung und/oder Augenbewegung des Probanden aufweist. In der gezeigten Ausführungsform weist das System 10 hierfür eine Kamera 18 auf, welche auf der Anzeigevorrichtung 12 angeordnet ist. Alternativ können beispielsweise Bewegungssensoren verwendet werden. Auch eine elektrookulographische (EOG) Bestimmung der Augenbewegung ist möglich.

Des Weiteren kann optional vorgesehen sein, dass das System 10 einen exzentrischen Photorefraktor 19 aufweist. Der exzentrische Photorefraktor 19 kann auf der Anzeigevorrichtung 12 angeordnet sein. Vorzugsweise ist eine Kamera 18 vorgesehen, welche zum einen Teil des exzentrischen Photorefraktors 19 ist und ferner als Mittel zum Erfassen der Kopfbewegung und/oder Augenbewegung des Probanden dient. Es kann somit ein Synergieeffekt erzielt werden.

Fig. 2 eine Ausführungsform eines Verfahrens zur Bestimmung der subjektiven Refraktionseigenschaften eines Auges eines Probanden basierend auf der Verwendung eines natürlichen Bildes dargestellt. Das Verfahren ist allgemein mit 100 bezeichnet.

Das Verfahren 100 weist hierbei die folgenden Schritte auf: In Schritt 110 wird ein Systems 10 wie beispielhaft vorstehend unter Bezugnahme auf Fig. 1 beschrieben bereitgestellt. In Schritt 120 wird mindestens ein natürliches Bild 30, welches in einer Speichereinrichtung 11 gespeichert ist, auf einer Anzeigevorrichtung 12 angezeigt. Es ist dabei auch möglich, dass in der Speichereinrichtung ein Bild mit einer Mehrzahl von Bildbereichen gespeichert ist, wobei verschiedene Bildbereiche unterschiedliche Ortsfrequenzen aufweisen und wobei das natürliche Bild eine natürliche Szenerie abbildet und dieses Bild auf der Anzeigevorrichtung 12 angezeigt wird. In Schritt 130 werden verschiedene lichtbrechende Elemente 14, 15 in einen optischen Pfad zwischen dem Auge 20 des Probanden und der Anzeigevorrichtung 12 mittels einer Optikanordnung 13 eingestellt, wobei die Optikanordnung 13 in einem vorgegebenen Abstand d von der Anzeigevorrichtung 12 angeordnet ist. Für einen beispielhaften Ablauf der Refraktionsbestimmung wird erneut auf Fig. 11 verwiesen.

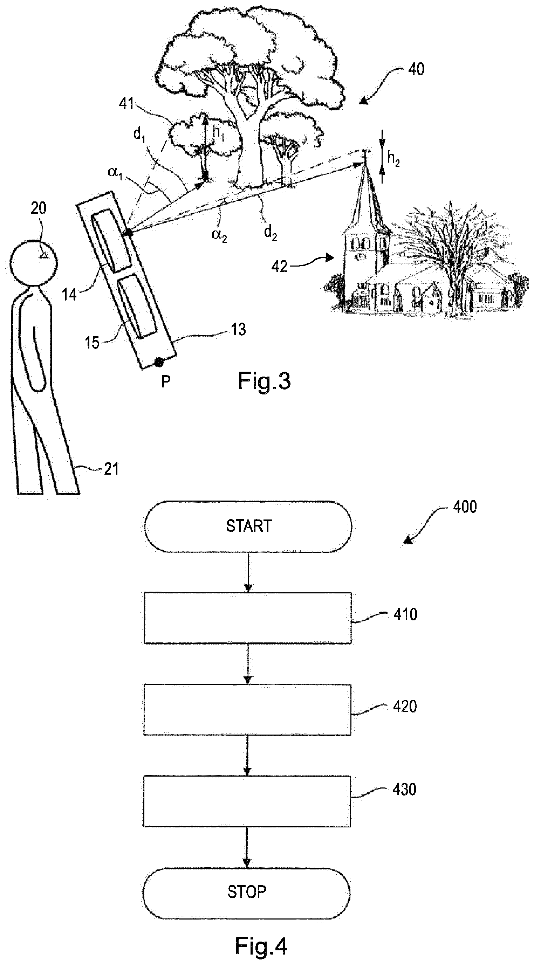

Fig. 3 zeigt ein beispielhaftes Szenario zur Bestimmung der subjektiven Refraktionseigenschaften eines Auges 20 eines Probanden 21 anhand einer natürlichen Szenerie 40.

Fig. 4 zeigt ein entsprechendes Verfahren 400 zur Bestimmung der subjektiven Refraktionseigenschaften eines Auges 20 eines Probanden 21 basierend auf der Verwendung einer natürlichen Szenerie 40, wobei das Verfahren die folgenden Schritte aufweist: In Schritt 410 wird eine Testumgebung, welche die natürliche Szenerie 40 aufweist bereitgestellt, wobei die natürliche Szenerie von einer vorgegebenen Position P aus betrachtet verschiedene Bereiche unterschiedlicher Ortsfrequenzen aufweist. In Schritt 420 wird eine Optikanordnung 13 zum Einstellen von verschiedenen lichtbrechenden Elementen 14, 15 in einen optischen Pfad zwischen dem Auge 20 des Probanden und der natürlichen Szenerie bereitgestellt. In Schritt 430 werden verschiedenen lichtbrechenden Elementen 14, 15 in den optischen Pfad zwischen dem Auge 20 des Probanden und der natürlichen Szenerie mittels der Optikanordnung 13 eingestellt, wobei die Optikanordnung 13 an der vorgegebenen Position P angeordnet ist. Für einen beispielhaften Ablauf der Refraktionsbestimmung wird erneut auf Fig. 11 verwiesen.

Die natürliche Szenerie 40 weist, wie auch ein natürliches Bild 30, Merkmale unterschiedlicher Strukturgröße auf, welche unterschiedlichen Sehwinkeln $\alpha_1$, $\alpha_2$ des Probanden 21 entsprechen. In dem in Fig. 3 dargestellten Beispiel weist die natürliche Szenerie 40 eine Gruppe von Bäumen unterschiedlicher Größe auf. Beispielsweise entspricht die Höhe h1 des Baumes 41 aus dem Betrachtungsabstand d1 betrachtet einem Sehwinkel $\alpha_1$. Wenn der Proband den Baum 41 nicht erkennen kann, so ist sein Auge zumindest ohne helfendes lichtbrechendes optisches Element 14, 15, nicht in der Lage den Winkel $\alpha_1$ aufzulösen. Es können testweise verschiedene lichtbrechende Elemente 14, 15 in den optischen Pfad zwischen dem Auge 20 des Probanden und dem Baum 41 eingebracht werden. So kann einen Refraktionsfehler des Auges des Probanden korrigiert und bestimmt werden, und der Proband ggf. die gewünschte Struktur, hier den baum 41 erkennen. Weitere Details sind unter Bezugnahme auf Fig. 11 beschrieben.

Das Vorgehen kann für weitere Strukturen bzw. Elemente der natürlichen Szenerie wiederholt werden. Vorzugsweise werden sukzessive kleinere Strukturen abgefragt. Dadurch erfolgt eine Annäherung an die Auflösungsschwelle des Probanden. Im Fall der Kirche 42 kann der Proband beispielsweise nacheinander gefragt werden, was für eine Art Gebäude abgebildet ist, wie viele Fenster der Turm aufweist bzw. was auf der Spitze des Turmes abgebildet ist. Wenn der Proband beispielsweise den Hahn auf der Kirchturmspitze erkennen kann, entspricht dies mit der Höhe des Hahnes $h_2$ aus dem Betrachtungsabstand $d_2$ einem Auflösungsvermögen von mindestens dem Sehwinkel $\alpha_2$. Mit anderen Worten kann die Bestimmung der subjektiven Refraktionseigenschaften nicht nur mit einer Anzeigevorrichtung, auf welcher ein natürliches Bild angezeigt wird, erfolgen, sondern unmittelbar indem eine natürliche Szenerie durch die Optikanordnung betrachtet wird. Die nachfolgenden Ausführungen zu natürlichen Bildern gelten entsprechend.

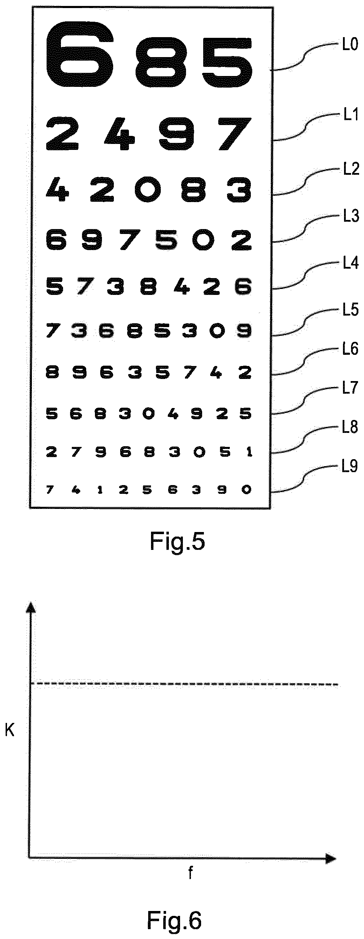

Fig. 5 zeigt ein Beispiel einer konventionellen Sehtesttafel. Als Sehtestzeichen kommen hierbei Zahlen zum Einsatz. Es sind jedoch auch andere Sehtestzeichen, wie sogenannte Landoltringe oder Snellen-Haken üblich. Die Sehtesttafel weist zehn Zeilen L0 bis L9 mit Zahlen unterschiedlicher Größen auf. In der Praxis werden immer die kompletten Einzeloptotypen für die Bestimmung der Sehschärfe benutzt. Im vorliegenden Beispiel werden also nacheinander und unabhängig voneinander einzelne Zahlen abgefragt.

Unabhängig von der Größe werden die Sehtestzeichen schwarz auf weißem Grund dargestellt. Ein entsprechendes schematisches Diagramm des Kontrastes gegenüber der Ortsfrequenz für konventionelle Sehtesttafeln ist in Fig. 6 dargestellt. Der Kontrast hat gemäß nationalen wie internationalen Normen sehr hoch zu sein, idealerweise nach Michelson gleich 1, und zwar bei allen getesteten Größen der Sehzeichen und somit bei allen verwendeten Ortsfrequenzen. Wie in Fig. 6 gezeigt ist der Kontrast konstant gegenüber der Ortsfrequenz und unabhängig von der Größe bei allen Sehtestzeichen gleich.

Bei Verwendung einer Sehtesttafel bzw. Sehprobentafel, wie beispielhaft in Fig. 5 gezeigt, werden die einzelnen Zeilen benutzt, um Refraktionsfehler des Auges und damit eine beste Brillenstärke zu definieren, welche einen vorhandenen Refraktionsfehler ausgleicht. Insbesondere wird bei Verwendung einer Sehtesttafel die Sehschärfe des Probanden mittels unterschiedlicher lichtbrechender Elemente der Optikanordnung in den einzelnen Reihen getestet und die lichtbrechenden Elemente werden dahingehend verändert, dass es dem Probanden möglich ist, eine möglichst kleine Reihe erkennen zu können. Wird die Sehtesttafel aus Fig. 5 bei einer Bildgröße von 998 Pixeln Höhe und 2120 Pixeln Breite in einer Entfernung von 1 m auf einem Bildschirm mit einer Pixelauflösung von 0.0275 Zentimeter pro Pixeln [cm/px] genutzt, ergeben sich die folgenden Sehschärfewerte für die Reihen L1-L9 gemäß Tabelle 1. Neben Angaben über die Pixeldimension ist das benötigte minimale Auflösungsvermögen [logMAR], um ein Detail erkennen zu können, angegeben.

TABELLE 1

| Zeile | Pixelhöhe der Zeichen | Minimal benötigtes Auflösungsvermögen [Sehschärfe] | Minimal benötigte Sehschärfe [logMAR] |
|---|---|---|---|
| L1 | 137 | 0.04 | 1.41 |
| L2 | 102 | 0.05 | 1.29 |
| L3 | 86 | 0.06 | 1.21 |
| L4 | 70 | 0.08 | 1.12 |
| L5 | 60 | 0.09 | 1.05 |
| L6 | 52 | 0.10 | 0.99 |
| L7 | 48 | 0.11 | 0.96 |
| L8 | 43 | 0.12 | 0.91 |
| L9 | 33 | 0.16 | 0.80 |

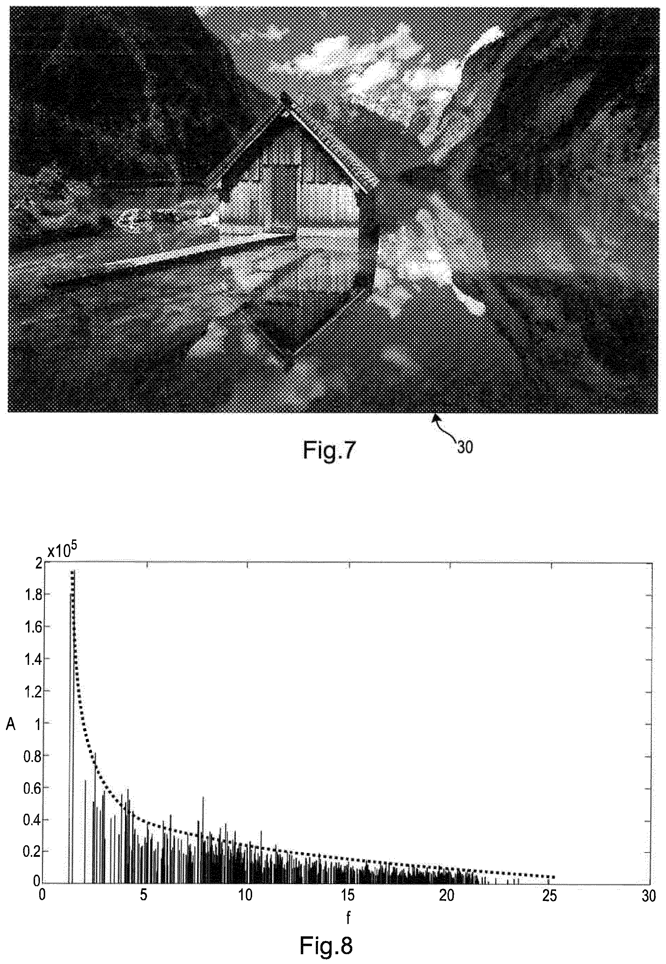

Im Gegensatz zu einer konventionellen Sehtesttafel zeigt Fig. 7 ein Beispiel für ein natürliches Bild 30. Im vorliegenden Beispiel entspricht das natürliche Bild 30 vorzugsweise einer Fotografie von einer typischen ländlichen Umgebung in der der Proband lebt. Es ist eine Hütte bzw. ein Bootshaus am Rande eines Sees vor dem Hintergrund von Bergen abgebildet.

Die Kenngrößen von natürlichen Bildern folgen bestimmten Gesetzmäßigkeiten. Die räumlichen Inhalte eines natürlichen Bildes lassen sich beispielsweise durch eine FourierTransformation mathematisch berechnen. Beispielsweise kann hierbei in der Software Matlab der Befehl FFT2 verwendet werden. In Abhängigkeit von der Pixeldichte auf einem Computermonitor und dem Betrachtungsabstand zu dem Monitor wird ermittelt, welche Ortsfrequenzen in einem Bild vorhanden sind.

Fig. 8 zeigt ein Ortsfrequenzdiagramm des Beispielbildes aus Fig. 7. Auf der horizontalen Achse ist die Ortsfrequenz f in Perioden pro Grad und auf der vertikalen Achse ist die Anzahl der entsprechenden Ortsfrequenzen A angegeben. Die Darstellung in Fig. 8 entspricht einer Ortsfrequenzanalyse bzw. Fourier-Transformation des Bildes aus Fig. 7 wenn es in einer Größe von 1920 Pixeln Breite und 1200 Pixeln Höhe in einem Abstand von 1 m bei einer Pixelauflösung von 0.0275 Zentimeter pro Pixel [cm/px] betrachtet wird. Fig. 6 gibt die Anzahl der unterschiedlichen Ortsfrequenzen für die y-Richtung, also die vertikale Richtung, der Abbildung in Fig. 5 an. Ein natürliches Bild kann mitunter dadurch gekennzeichnet sein, dass die Anzahl der Ortsfrequenzen bzw. eine Amplitude des Ortsfrequenzdiagramms mit steigender Ortsfrequenz abnimmt. Diese Tendenz ist in Fig. 8 durch die gestrichelte Linie dargestellt. Insbesondere ist die Amplitude des Ortsfrequenzdiagramms bei einem natürlichen Bild umgekehrt proportional zur Ortsfrequenz (siehe auch Tolhurst et al. "Amplitude spectra of natural images", Ophthal. Physiol. Opt., Vol. 12, 1992 sowie Field et al. "Relations between the statistics of natural images and the response properties of cortical cells", Vol. 4, No. 12, Journal of the Optical Society of America, 1987).

Bei der Verwendung einer konventionellen Sehtesttafel, wie in Fig. 5, werden die einzelnen Zeilen mit einzelnen Sehtestzeichen zur Bestimmung der subjektiven Refraktionseigenschaften bzw. der Brillenstärke benutzt. Demgegenüber können bei Verwendung eines natürlichen Bildes, wie in Fig. 7, verschiedene Bildbereiche bzw. verschiedene Bildinhalte verwendet werden. Durch eine Ortsfrequenzanalyse kann vorzugsweise auf den Bildinhalt geschlossen werden. Aus der Ortsfrequenz von Merkmalen bzw. Bildinhalten in dem natürlichen Bild kann eine Transformation auf die Sehschärfe erfolgen.

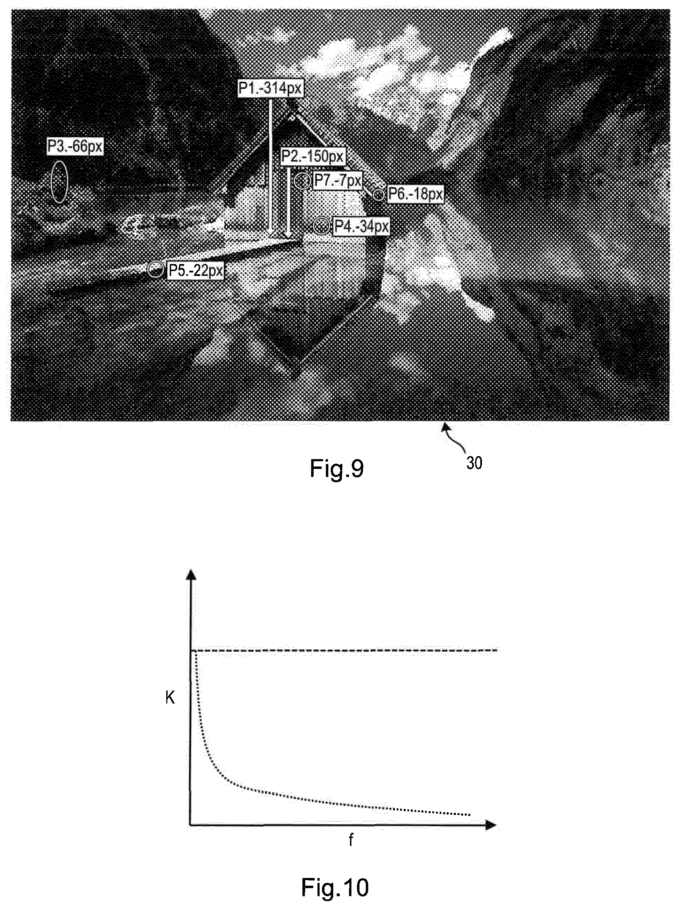

Für eine bessere Darstellung zeigt Fig. 9 nochmals das Beispielbild 30 aus Fig. 7 mit Kennzeichnung von Bereichen unterschiedlicher Ortsfrequenzen. Dabei sind unterschiedliche Details des Bildes durch Positionen im Bild P1 bis P7 definiert. Dabei sind auch die entsprechenden Pixeldimensionen durch ihre Pixelanzahl für die unterschiedlichen beispielhaft ausgewählten Details angegeben. Bei den ausgewählten Details kann es sich um sogenannte Regions of interest (ROIs) handeln, welche zur Befragung des Probanden bei der habituellen Refraktionsbestimmung verwendet werden.

Die nachfolgende beispielhafte Tabelle gibt an wie hoch bei einer Bildgröße von 1920 Pixeln Breite und 1200 Pixeln Höhe für eine Entfernung von einem Meter und eine Pixelauflösung der Anzeigevorrichtung 12 von 0.0275 Zentimeter pro Pixel [cm/px] die Sehschärfe des Auges sein muss, um die Details erkennen zu können.

TABELLE 2

| Position | Pixelanzahl | Minimal benötigtes Auflösungsvermögen [Sehschärfe] | Minimal benötigte Sehschärfe [logMAR] |
|---|---|---|---|
| P1 | 314 | 0.0158 | 1.8 |
| P2 | 150 | 0.315 | 1.5 |
| P3 | 66 | 0.08 | 1.10 |
| P4 | 34 | 0.16 | 0.80 |
| P5 | 22 | 0.25 | 0.6 |
| P6 | 18 | 0.3 | 0.5 |
| P7 | 7 | 0.8 | 0.1 |

Fig. 10 zeigt ein schematisches Diagramm des Kontrastes K gegenüber der Ortsfrequenz f für ein Bild gemäß der vorliegenden Offenbarung. Im Gegensatz zu einer konventionellen Sehtesttafel, welche einen konstanten Kontrast gegenüber der Ortsfrequenz aufweist, wird in der vorliegenden Offenbarung insbesondere die Verwendung von Bildern zur Refraktionsbestimmung vorgeschlagen, bei welchen der Kontrast mit zunehmender Ortsfrequenz abnimmt. Zur Bestimmung des Kontrasts kann der Grauwert eines Pixels mit dem Grauwert seines Nachbarpixels verglichen werden. Insbesondere kann der Kontrast umgekehrt proportional zur Ortsfrequenz sein.

Mit anderen Worten liegt eine weitere Gesetzmäßigkeit für die vorgeschlagenen natürlichen Bilder in der Abnahme des Kontrasts mit zunehmender Ortsfrequenz. Bei der Auswertung von unterschiedlichen natürlichen Bildern konnten beispielsweise Tollhurst et al. (Tolhurst, D. J., Tadmor, Y., & Chao, T. (1992). Amplitude spectra of natural images. Ophthalmic and Physiological Optics, 12(2), 229-232.) zeigen, dass die Steigung der Abnahme mit zunehmender Ortsfrequenz im Mittel -1.2 beträgt. Dieser Zusammenhang wird auch von Burton et. al. (Burton et. al., "Color and spatial structure in natural scenes", Applied Optics, Vol. 26, No. 1, 1987) beschrieben.

Fig. 11 zeigt eine beispielhafte Ausführungsform eines Verfahrens zur Bestimmung der subjektiven Refraktionseigenschaften eines Auges 20 eines Probanden. Die Refraktionsbestimmung kann unter Verwendung des natürlichen Bildes aus Fig. 7 bzw. Fig. 9, bzw. in einem Szenario mit einer natürlichen Szenerie wie in Fig. 3, wie folgt durchgeführt werden:

In Schritt 210 kann zunächst eine erforderliche Sphäre des Probanden bestimmt werden. Der Proband trägt keine eigene Korrektion und betrachtet das auf der Anzeigevorrichtung 12 dargestellte natürliche Bild 30 durch die Optik 13, hier durch einen Phoropter. Beispielsweise soll der Proband zunächst Detail gemäß Position 1 (Haus) erkennen. Kann er dieses nicht, wird zuerst ein Plusglas (nach Stufungstabelle) vorgehalten und gefragt, ob es schlechter wird. Wenn nein, kann weiter mit Pluslinsen korrigiert werden, bis ein zufriedenstellendes Ergebnis erzielt wird. Wenn ja, kann mit Minusgläsern weiter korrigiert werden. Nachfolgenden können nun Gläser nach Stufungstabelle verwendet und immer kleiner Details aus dem Bild verwendet. Wenn der Kunde beispielsweise Position 7 (Türbeschlag) im Bild erkennen kann ist das gewünschte bzw. beste sphärische Glas gefunden.

Die nachfolgende Tabelle zeigt eine Stufungstabelle für sphärische Gläser. Die Stufungstabelle gibt eine Abstufung der vorzuhaltenden bzw. vorzuschaltenden Gläser bei der Bestimmung sphärischer Korrektionen in Abhängigkeit vom Visus an.

TABELLE 3

| Visus | Glasabstufung in Dioptrien (dpt) |
|---|---|
| unter 0,05 | 2 dpt |
| 0,05 bis 0,2 | 1 dpt |
| 0,2 bis 0,5 | 0,5 dpt |
| über 0,5 | 0,25 dpt |

In Schritt 220 kann eine Bestimmung einer astigmatischen Korrektion erfolgen. Nach dem das beste sphärische Glas gefunden wurde, erfolgt die Prüfung auf Astigmatismus. Der Proband kann beispielsweise gebeten werden Position 5 zu betrachten. Dann wird ein Kreuzzylinder (Abstufung nach Stufungstabelle) eingebracht und gefragt ob es besser oder schlechter wird. Es kann also erneut eine Vorhaltebefragung durchgeführt werden. Je nach Antwort wird ein Astigmatismus bestimmt und eine entsprechende Korrektur eingebracht (ja) oder nicht (nein). Es kann eine weitere Vorhaltebefragung erfolgen, bis es keine Verbesserung bzw. eine Verschlechterung mit der Vorhaltebefragung gibt. Dabei ist darauf zu achten, dass der sphärische Fehler nachgezogen wird (nach Stufungstabelle).

In Schritt 230 kann eine Bestimmung einer Achslage der astigmatischen Korrektion erfolgen. Hierbei kann mittels Wendebefragung die genaue Achslage des korrigierenden Zylinderglases gefunden werden. Der Probanden wird dabei beispielsweise gebeten auf Position 3 (Pflanze) zu sehen. Die Wendebefragung kann so lange durchgeführt werden, bis der Kunde keinen Unterschied zwischen den beiden Achslagen bei der Wendebefragung sieht.

In Schritt 240 kann ein monokularer sphärischer Feinabgleich erfolgen, insbesondere nach Durchführung der Schritte 210 bis 230. Hierfür kann der Proband gebeten werden, Position 6 zu betrachten. Es können Plusgläser oder Minusgläser vorgehalten werden, bis der höchste Visus, also die größte Sehschärfe, mit maximalem Plus erreicht wird.

In Schritt 250 kann eine Refraktionsbestimmung des zweiten Auges analog zu den vorstehend beschriebenen Schritten 210 bis 240 erfolgen.

In Schritt 260 kann ein binokularer sphärischer Feinabgleich erfolgen. Hierfür kann Schritt 240 unter binokularen Bedingungen durchgeführt werden. Dazu kann der Kunde zum Beispiel die Wolken am Himmel betrachten.

Optional kann eine Bestimmung von Messwerten aus einer Mess- und Korrektionsmethode nach Haase mit bisher bekannten Tests im Anschluss an die Ermittlung von Refraktionsfehlern niederer Ordnung, wie Sphäre, Astigmatismus und Zylinder, umgesetzt werden.

**Patentansprüche**

1. System (10) zur Bestimmung der subjektiven Refraktionseigenschaften eines Auges (20) eines Probanden basierend auf der Verwendung eines natürlichen Bildes (30), wobei das System Folgendes aufweist:

   - eine Speichereinrichtung (11), in welcher mindestens ein natürliches Bild (30) gespeichert ist;
   - eine Anzeigevorrichtung (12) zum Anzeigen des mindestens einen natürlichen Bildes (30) aus der Speichereinrichtung (11); und
   - eine Optikanordnung (13) zum Einstellen von verschiedenen lichtbrechenden Elementen (14, 15) in einen optischen Pfad zwischen dem Auge (20) des Probanden und der Anzeigevorrichtung (12), wobei die Optikanordnung (13) in einem vorgegebenen Abstand (d) von der Anzeigevorrichtung (12) angeordnet ist, wobei das natürliche Bild (30) mindestens zwei Bereiche mit unterschiedlichen Ortsfrequenzen (f) aufweist; **gekennzeichnet durch** Mittel (18) zum Erfassen der Augenbewegung des Probanden, wobei das System (10) eingerichtet ist über Analyse einer Verteilung von Augenbewegungen auf dem natürlichen Bild nachzuvollziehen, welche Ortsfrequenzen häufig angeblickt werden.

2. System nach Anspruch 1, **dadurch gekennzeichnet, dass** das natürliche Bild einen ortsfrequenzabhängigen Kontrast (K) aufweist, wobei der Kontrast mit zunehmender Ortsfrequenz (f) abnimmt.

3. System nach Anspruch 2, **dadurch gekennzeichnet, dass** der Kontrast (K) umgekehrt proportional zur Ortsfrequenz (f) ist.

4. System nach einem der vorhergehenden Ansprüche, **dadurch gekennzeichnet, dass** der Kontrast (K) des natürlichen Bildes für höhere Ortsfrequenzen angehoben wird, insbesondere auf ein Kontrastniveau einer durch das natürliche Bild abgebildeten natürlichen Szenerie.

5. System nach einem der vorhergehenden Ansprüche, **dadurch gekennzeichnet, dass** das natürliche Bild (30) eine Verteilung von Ortfrequenzen (f) aufweist, welche eine Mehrzahl, vorzugsweise alle, zur Bestimmung von subjektiven Refraktionseigenschaften des Auges (20) des Probanden erforderlichen Ortsfrequenzen in einem einzigen Bild umfassen.

6. System nach einem der vorhergehenden Ansprüche, **dadurch gekennzeichnet, dass** das natürliche Bild (30) Strukturen mit unterschiedlichen Ortsfrequenzen (f) zeigt, wobei die Ortsfrequenzverteilung, bei Betrachtung aus dem vorgegebenen Abstand (d), mindestens eine Ortsfrequenz kleiner oder gleich 0,3 und mindestens eine Ortsfrequenz größer oder gleich 60 Perioden pro Grad aufweist, vorzugsweise mindestens eine Ortsfrequenz kleiner oder gleich 0,01 und mindestens eine Ortsfrequenz größer oder gleich 80 Perioden pro Grad aufweist.

7. System nach einem der vorhergehenden Ansprüche, **gekennzeichnet durch** eine Auswahleinrichtung zum Auswählen des natürlichen Bildes (30) gemäß Präferenzen des Probanden.

8. System nach einem der vorhergehenden Ansprüche, **dadurch gekennzeichnet, dass** das natürliche Bild (30) mindestens eine Verfremdung aufweist.

9. System nach einem der vorhergehenden Ansprüche, **dadurch gekennzeichnet, dass** wenigstens ein Sehzeichen zumindest teilweise in das natürliche Bild (30) integriert ist.

10. System nach einem der vorhergehenden Ansprüche, **dadurch gekennzeichnet, dass** die Anzeigevorrichtung (12) eine gekrümmte Anzeigefläche aufweist.

11. System nach einem der vorhergehenden Ansprüche, **dadurch gekennzeichnet, dass** das natürliche Bild (30) ein Video ist.

12. System nach einem der vorhergehenden Ansprüche, **dadurch gekennzeichnet, dass** das System (10) ferner einen exzentrischen Photorefraktor (19) aufweist.

13. Verfahren (100) zur Bestimmung der subjektiven Refraktionseigenschaften eines Auges (20) eines Probanden basierend auf der Verwendung eines natürlichen Bildes (30), wobei das Verfahren die folgenden Schritte aufweist:

   - Bereitstellen eines Systems (10) zur Bestimmung der subjektiven Refraktionseigenschaften eines Auges (20) eines Probanden basierend auf der Verwendung eines natürlichen Bildes (30), wobei das System Folgendes aufweist:

      - eine Speichereinrichtung (11), in welcher mindestens ein natürliches Bild (30) gespeichert ist;
      - eine Anzeigevorrichtung (12) zum Anzeigen des mindestens einen natürlichen Bildes (30) aus der Speichereinrichtung (11);
      - eine Optikanordnung (13) zum Einstellen von verschiedenen lichtbrechenden Elementen (14, 15) in einen optischen Pfad zwischen dem Auge (20) des Probanden und der Anzeigevorrichtung (12);

   - Anzeigen des mindestens einen natürlichen Bildes (30), welches in einer Speichereinrichtung (11) gespeichert ist, auf der Anzeigevorrichtung (12); und
   - Einstellen von verschiedenen lichtbrechenden Elementen (14, 15) in den optischen Pfad zwischen dem Auge

(20) des Probanden und der Anzeigevorrichtung (12) mittels der Optikanordnung (13), wobei die Optikanordnung (13) in einem vorgegebenen Abstand (d) von der Anzeigevorrichtung (12) angeordnet ist;

wobei das natürliche Bild (30) mindestens zwei Bereiche mit unterschiedlichen Ortsfrequenzen (f) aufweist;

**gekennzeichnet durch**

- Erfassen der Augenbewegung des Probanden, und
- Nachvollziehen, über Analyse einer Verteilung von Augenbewegungen auf dem natürlichen Bild, welche Ortsfrequenzen häufig angeblickt werden.

## Claims

1. System (10) for determining the subjective refractive properties of an eye (20) of a subject based on the use of a natural image (30), wherein the system includes the following:

   - a storage device (11) in which at least one natural image (30) is stored;
   - a display apparatus (12) for displaying the at least one natural image (30) from the storage device (11); and
   - an optics arrangement (13) for placing different light-refractive elements (14, 15) into an optical path between the eye (20) of the subject and the display apparatus (12), wherein the optics arrangement (13) is arranged at a specified distance (d) from the display apparatus (12), wherein the natural image (30) has at least two regions having different spatial frequencies (f); **characterized by** means (18) for capturing the eye movement of the subject, wherein the system (10) is set up to track which spatial frequencies are frequently looked at by analysing a distribution of eye movements on the natural image.

2. System according to Claim 1, **characterized in that** the natural image has a spatial-frequency-dependent contrast (K), wherein the contrast decreases as the spatial frequency (f) increases.

3. System according to Claim 2, **characterized in that** the contrast (K) is inversely proportional to the spatial frequency (f).

4. System according to one of the preceding claims, **characterized in that** the contrast (K) of the natural image is raised for higher spatial frequencies, in particular to a contrast level of a natural scenery imaged by the natural image.

5. System according to one of the preceding claims, **characterized in that** the natural image (30) has a distribution of spatial frequencies (f) comprising a plurality of, preferably all, spatial frequencies that are necessary for determining subjective refraction properties of the eye (20) of the subject in a single image.

6. System according to one of the preceding claims, **characterized in that** the natural image (30) shows structures having different spatial frequencies (f), wherein the spatial frequency distribution has, under observation from the specified distance (d), at least one spatial frequency lower than or equal to 0.3 and at least one spatial frequency greater than or equal to 60 cycles per degree, preferably at least one spatial frequency lower than or equal to 0.01 and at least one spatial frequency greater than or equal to 80 cycles per degree.

7. System according to one of the preceding claims, **characterized by** a selection device for selecting the natural image (30) in accordance with preferences of the subject.

8. System according to one of the preceding claims, **characterized in that** the natural image (30) has at least one alien component.

9. System according to one of the preceding claims, **characterized in that** at least one vision symbol is integrated at least partially into the natural image (30) .

10. System according to one of the preceding claims, **characterized in that** the display apparatus (12) has a curved display surface.

11. System according to one of the preceding claims, **characterized in that** the natural image (30) is a video.

12. System according to one of the preceding claims, **characterized in that** the system (10) furthermore has an eccentric photorefractor (19).

**13.** Method (100) for determining the subjective refraction properties of an eye (20) of a subject based on the use of a natural image (30), wherein the method includes the following steps:

- providing a system (10) for determining the subjective refraction properties of an eye (20) of a subject based on the use of a natural image (30), wherein the system includes the following:

  - a storage device (11) in which at least one natural image (30) is stored;
  - a display apparatus (12) for displaying the at least one natural image (30) from the storage device (11) ;
  - an optics arrangement (13) for placing different light-refractive elements (14, 15) in an optical path between the eye (20) of the subject and the display apparatus (12);

- displaying the at least one natural image (30) that is stored in a storage device (11) on the display apparatus (12); and
- placing different light-refractive elements (14, 15) into the optical path between the eye (20) of the subject and the display apparatus (12) by way of the optics arrangement (13), wherein the optics arrangement (13) is arranged at a specified distance (d) from the display apparatus (12);

  wherein the natural image (30) has at least two regions having different spatial frequencies (f);
  **characterised by**

- Capturing the eye movement of the subject, and
- Tracking, by analysing the distribution of eye movements on the natural image, which spatial frequencies are frequently looked at.

**Revendications**

**1.** Système (10) pour déterminer les propriétés de réfraction subjectives d'un œil (20) d'un sujet sur la base de l'utilisation d'une image naturelle (30), le système présentant les éléments suivants :

- un dispositif de mémoire (11) dans lequel est mémorisée au moins une image naturelle (30) ;
- un dispositif d'affichage (12) pour afficher l'au moins une image naturelle (30) issue du dispositif de mémoire (11) ; et
- un agencement d'optique (13) pour ajuster différents éléments de réfraction (14, 15) dans un chemin optique entre l'œil (20) du sujet et le dispositif d'affichage (12), l'agencement d'optique (13) étant disposé à une distance prédéfinie (d) du dispositif d'affichage (12), l'image naturelle (30) présentant au moins deux régions de fréquences spatiales différentes (f) ;

**caractérisé par** des moyens (18) pour détecter le mouvement des yeux du sujet, le système (10) étant conçu pour appréhender, par l'analyse d'une distribution de mouvements des yeux sur l'image naturelle, quelles fréquences spatiales sont regardées fréquemment.

**2.** Système selon la revendication 1, **caractérisé en ce que** l'image naturelle présente un contraste (K) dépendant de la fréquence spatiale, le contraste diminuant avec l'augmentation de la fréquence spatiale (f) .

**3.** Système selon la revendication 2, **caractérisé en ce que** le contraste (K) est inversement proportionnel à la fréquence spatiale (f).

**4.** Système selon l'une quelconque des revendications précédentes, **caractérisé en ce que** le contraste (K) de l'image naturelle est augmenté pour des fréquences spatiales plus élevées, en particulier à un niveau de contraste d'un paysage naturel reproduit par l'image naturelle.

**5.** Système selon l'une quelconque des revendications précédentes, **caractérisé en ce que** l'image naturelle (30) présente une distribution de fréquences spatiales (f) qui comprennent une pluralité, de préférence la totalité, des fréquences spatiales nécessaires pour déterminer des propriétés de réfraction subjectives de l'œil (20) du sujet dans une seule image.

**6.** Système selon l'une quelconque des revendications précédentes, **caractérisé en ce que** l'image naturelle (30) montre des structures avec des fréquences spatiales différentes (f), la distribution de fréquences spatiales, en regardant depuis la distance prédéfinie (d), présentant au moins une fréquence spatiale inférieure ou égale à 0,3 et au moins une fréquence spatiale supérieure ou égale à 60 périodes par degré, de préférence au moins une fréquence spatiale inférieure ou égale à 0,01 et au moins une fréquence spatiale supérieure ou égale à 80 périodes par degré.

7. Système selon l'une quelconque des revendications précédentes, **caractérisé par** un dispositif de sélection pour sélectionner l'image naturelle (30) selon des préférences du sujet.

8. Système selon l'une quelconque des revendications précédentes, **caractérisé en ce que** l'image naturelle (30) présente au moins une distanciation.

9. Système selon l'une quelconque des revendications précédentes, **caractérisé en ce qu'**au moins un signe visuel est intégré au moins en partie dans l'image naturelle (30).

10. Système selon l'une quelconque des revendications précédentes, **caractérisé en ce que** le dispositif d'affichage (12) présente une surface d'affichage courbe.

11. Système selon l'une quelconque des revendications précédentes, **caractérisé en ce que** l'image naturelle (30) est une vidéo.

12. Système selon l'une quelconque des revendications précédentes, **caractérisé en ce que** le système (10) présente en outre un photoréfracteur excentrique (19).

13. Procédé (100) pour déterminer les propriétés de réfraction subjectives d'un œil (20) d'un sujet sur la base de l'utilisation d'une image naturelle (30), le procédé présentant les étapes suivante :

   - la fourniture d'un système (10) pour déterminer les propriétés de réfraction subjectives d'un œil (20) d'un sujet sur la base de l'utilisation d'une image naturelle (30), le système présentant les éléments suivants :

      - un dispositif de mémoire (11) dans lequel est mémorisée au moins une image naturelle (30) ;
      - un dispositif d'affichage (12) pour afficher l'au moins une image naturelle (30) issue du dispositif de mémoire (11) ;
      - un agencement d'optique (13) pour ajuster différents éléments de réfraction (14, 15) dans un chemin optique entre l'œil (20) du sujet et le dispositif d'affichage (12) ;

   - l'affichage d'au moins une image naturelle (30) qui est mémorisée dans un dispositif de mémoire (11), sur le dispositif d'affichage (12) ; et
   - l'ajustement de différents éléments de réfraction (14, 15) dans le chemin optique entre l'œil (20) du sujet et le dispositif d'affichage (12) au moyen de l'agencement d'optique (13), l'agencement d'optique (13) étant disposé à une distance prédéfinie (d) du dispositif d'affichage (12) ;

      dans lequel l'image naturelle (30) présente au moins deux régions de fréquences spatiales différentes (f) ;
      **caractérisé par**

   - la détection du mouvement des yeux du sujet, et
   - le fait d'appréhender, par l'analyse d'une distribution de mouvements des yeux sur l'image naturelle, quelles fréquences spatiales sont regardées fréquemment.

10

18,19

d

20

14

15

13

12  11

30

## Fig.1

100

START

110

120

130

STOP

## Fig.2

Fig.3

Fig.4

685 — L0

2 4 9 7 — L1

4 2 0 8 3 — L2

6 9 7 5 0 2 — L3

5 7 3 8 4 2 6 — L4

7 3 6 8 5 3 0 9 — L5

8 9 6 3 5 7 4 2 — L6

5 6 8 3 0 4 9 2 5 — L7

2 7 9 6 8 3 0 5 1 — L8

7 4 1 2 5 6 3 9 0 — L9

Fig.5

Fig.6

Fig.7

Fig.8

Fig.9

Fig.10

Fig.11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6325513 B
- WO 2017050935 A1
- DE 102004055754 A1
- DE 4091126 C2
- EP 2842479 A1

**In der Beschreibung aufgeführte Nicht-Patentliteratur**

- **MASAOKA et al.** *Sensation of Realness From High-Resolution Images of Real Objects*
- **BEX. et al.** Spatial frequency, phase, and the contrast of natural images. *Journal of the Optical Society of America,* 2002, vol. 19 (6
- **TOLHURST et al.** Amplitude spectra of natural images. *Ophthal. Physiol. Opt.,* 1992, vol. 12
- **FIELD et al.** Relations between the statistics of natural images and the response properties of cortical cells. *Journal of the Optical Society of America,* 1987, vol. 4 (12
- **TOLHURST, D. J. ; TADMOR, Y. ; CHAO, T.** Amplitude spectra of natural images. *Ophthalmic and Physiological Optics,* 1992, vol. 12 (2), 229-232
- **BURTON.** Color and spatial structure in natural scenes. *Applied Optics,* 1987, vol. 26 (1